# EUROPEAN PATENT SPECIFICATION

(11) **EP 2 410 980 B1**
(45) Date of publication and mention of the grant of the patent: **13.12.2017**
(21) Application number: 10712229.3
(22) Date of filing: 26.03.2010
(51) Int. Cl.: A61Q 5/02, A61Q 5/12, A61K 8/73, A61K 8/86, A61K 8/892

(54) **AMINATED POLYMERS AND THEIR USE IN WATER-BORNE COMPOSITIONS**
AMINIERTE POLYMERE UND IHRE VERWENDUNG IN DURCH WASSER ÜBERTRAGENEN ZUSAMMENSETZUNGEN
POLYMÈRES AMINÉS ET LEUR UTILISATION DANS DES COMPOSITIONS AQUEUSES

(30) Priority: 27.03.2009 US 163974 P
(43) Date of publication of application: 01.02.2012
(73) Proprietor: Hercules Incorporated, Wilmington, DE 19808 (US)
(72) Inventor: SAU, Arjun C., Newark DE 19711 (US)
(74) Representative: Bülle, Jan
(86) International application number: PCT/US2010/028803
(87) International publication number: WO 2010/111576

(56) References cited:
- EP-A2- 0 880 962
- WO-A1-02/10256
- WO-A1-97/46211
- WO-A1-2006/133845
- DE-A1- 19 514 630
- DE-A1-102004 035 870
- FR-A1- 2 748 203
- FR-A1- 2 767 473
- US-B1- 6 649 155
- BRONNSACK ET AL: "Ein neuentwickeltes Vinylpyrrolidon-Harz für kosmetische Produkte /// A newly-developed vinylpyrrolidone resin for cosmetic products", RIECHSTOFFE, AROMEN, KOSMETICA,, vol. 29, no. 10, 1 January 1979 (1979-01-01), pages 206-207, XP009152462,

## Description

### FIELD OF INVENTION

The present invention relates to processes for the manufacture of aminated or amino-functionalized polymers and their use in water-borne compositions. More specifically, this invention relates to personal care compositions containing aminated polymers and one or more other conditioning agents, surfactants and other active or inactive ingredients.

### BACKGROUND OF THE INVENTION

To modify the properties of hair and skin to improve their condition or state of being, consumers use a wide variety of personal care products. These products comprise various surfactants and chemical additives collectively referred to as conditioners or conditioning agents. Examples of personal care products include hair care products, skin care products, deodorants, antiperspirants, lotions, creams and others.

For personal care products, there is a growing demand from consumers to have products designed specifically for their distinctive hair or skin and grooming habits. Consumers desire to have a single product for providing both cleansing and conditioning to hair or skin. For this reason, personal care compositions having such dual features continue to grow in popularity. One such personal care composition is shampoo. Shampoos, commonly referred to as "two-in-one" are extensively used by consumers for cleansing and conditioning hair.

Many factors affect the conditioning of hair. These factors include surface characteristics and morphology of the hair, the type of conditioner and the amount of the conditioner deposited on the hair during the cleansing process. The amount of the conditioner(s) deposited on the hair depends on *inter alia* the surface chemistry of the hair, chemical nature of the conditioner(s), the type and amount of detersive surfactants present in shampoo compositions and the pH of the compositions. The mechanism of retention of conditioners on hair is not comprehensively understood. It is, however, believed that conditioners are retained on the hair by a combination of Coulombic attractions and van der Waals forces.

In many commercial shampoos, zinc compounds, such as zinc pyrithione and zinc carbonate are included in the formulation to provide anti-dandruff functionality. Dandruff is due to the excessive shedding of dead skin cells from the scalp accompanied by tightness of scalp, dry feel irritation, itchiness, and flaking. It is primarily caused by fungi of the genus *Malassezia.* Anti-dandruff agents are well known as set forth in U. S. Patent No. 6,649,155. Also, anti-dandruff shampoos that also provide conditioning are well known and are disclosed in U. S. Patent No. 5,624,666.

Due to various chemical and mechanical treatments performed on hair, the chemical and morphological characteristics of the hair surface can significantly change. Chemical treatments leading to hair damage include bleaching, coloring, exposure to ultraviolet light, chemical relaxation or lanthonization, permanent hair waving, and styling. Mechanical treatments occasioning hair damage include combing and blow drying. In addition, a person's diet could also be a factor contributing to hair damage.

Chemical treatments can remove the outermost hydrophobic layer of hair (cuticle) and can damage the next layer. The hair surface naturally is negatively charged to a degree. However, with continual damage, the hair surface develops more negative charges, turns hydrophilic, becomes rough and tends to adhere. These changes, in turn, increase the friction and adhesion of hair making it difficult to comb. In addition, the damaged hair develops a flyaway look and tends to entangle. The end result is hair with an unhealthy and dull appearance.

During common styling practices such as blow drying, curling or straightening with irons, and use of other heat-based styling appliances, hair is subjected to high temperatures and hence, can undergo damage. Therefore, consumers need products that protect hair from damage during such treatments and condition damaged hair.

The main objectives of using conditioners are to mitigate the damages caused on the hair surface and to impart an improved feel to the hair. Treatment of hair with conditioners can dramatically change its surface chemistry and morphology leading to reduced friction and improved soft feel to the touch.

Originally conditioners were used to control the static charges on hair to eliminate the flyaway look after washing and drying the hair. Subsequently, conditioners were included in "two-in-one" shampoos to gain other benefits, such as healthy and shiny appearance, soft feel to the touch and easy to comb in the wet and dry state. Other benefits desired from conditioners are to maintain body and bounce and to repair split hair.

Typical conditioners used in shampoos are cationic surfactants, cationic polymers, nucleic acids, lipids, silicones, hydrocarbon oil, fatty esters or combinations thereof. Silicones have gained significant popularity as conditioners in many personal care compositions. Silicones are thermally stable liquids, spread easily on hair producing a protective film and help prevent water loss. They provide lubricity, shine and improved soft feel to the touch relative to compositions containing no conditioner. Because of these features, silicones have become the materials of choice to condition hair and are extensively used in commercial shampoos.

Chemically, silicones are polydimethylsiloxanes based on alternating silicon and oxygen atoms with two methyl groups attached to each silicon atom. A wide variety of silicones are commercially available from silicone manufactures, such as Dow Corning, GE Silicones, Wacker Chemicals Corporation and others. Certain silicones may contain other functional groups, such as amino, ammonium, hydroxyl, epoxy or polyalkylene glycols. Since silicones are water-insoluble, they are generally supplied as aqueous microemulsions to facilitate their incorporation into shampoo formulations and prevent phase separation on storage.

During the shampooing process, loss of a portion of the conditioner is very likely. However, loss of a large proportion of the conditioner can provide very little or no conditioning benefit at all. In such situations, to mitigate the conditioner loss during cleansing, high levels of the conditioner(s) need to be incorporated into the formulation to achieve the desired conditioning effect. Regrettably, the use of high levels of conditioners is undesirable as it i) increases the cost of manufacturing the personal care products, ii) could reduce lathering during applications and iii) could destabilize the formulation.

The presence of detersive surfactants in personal care compositions can also affect the degree of deposition of the conditioner on the hair. The function of the surfactants is to remove grease, oil, and particulates adsorbed onto hair or skin. Since silicones are hydrophobic or oil-like materials, they can be removed by surfactants during the cleansing process.

Cationic polymers have been used to provide conditioning to hair. Cationic polymers used in shampoos could be synthetic or natural polymers based. Natural polymer based cationic polymers, currently used in shampoos include cationic starch, cationic hydroxyethylcellulose and cationic guar. However, cationic polymers are difficult to remove from the surface of the negatively charged keratinous substrates, such as hair and skin, by washing. As a result, after repeated treatments with a shampoo, cationic polymers and other ingredients present in the shampoo tend to buildup on the hair surface making the hair look dull, unclean and less manageable. The effects of buildup of cationic polymers has been noted, (*Removal of cationic buildup from keratin surfaces by sodium polystyrene sulfonate,* B. Schweid et al., presented at PCIA, Shanghai, March 2002; US Patent No. 7,067,499). By lowering the cationic degree of substitution of cationically modified hydroxyethylcellulose, the buildup of cationic polymers on the surface of the hair can be reduced. However such cationic polymers do not provide broad surfactant compatibility ("Cationic conditioners that revitalize hair and skin", Amerchol Product Literature, WSP801, July 1998).

WO 2006/133845 discloses guar gum derivatives containing amino alkyl groups and a method for producing said polysaccharide derivatives by the etherification of free hydroxyl groups of guar gum or guar gum derivatives using dialkylaminoalkyl halides.

Since improvement in combing after shampooing is perceived by customers as the hair being in a better condition, the ease of combing hair in the wet and dry state is measured. Experimentally, the combing forces to drag the comb through wet and dry hair are measured. Shampoos containing commercial cationic polymers typically reduce the wet comb energy by 30-50% relative to shampoos containing no cationic polymers or other conditioners.

For silicone containing shampoos, cationic polymers have been found to enhance deposition of silicones onto hair. Since they aid in depositing silicones, cationic polymers are referred to as "cationic deposition polymers". The recent trend in shampoo formulations was to use a combination of cationic polymer(s) and silicones to achieve adequate conditioning as in U.S. Patent No. 6,649,155. By including silicones that are nonionic, some of the disadvantages, such as buildup problems due to the cationic polymer mentioned above, are eliminated.

Accordingly, there is a need for alternative polymers that provide adequate conditioning to keratinous substrates with very little or no silicones and yet be removed from keratinous substrates during a subsequent cleansing step. Shampoo formulators are challenged to develop high-performance, multifunctional products to meet unique consumers' needs based on hair structure and styling techniques. So far as the type of polymer is concerned, there is a preference for the use of natural components, including natural polymers, in personal care formulations.

When an amount, concentration, or other value or parameter is given as either a range, preferred range, or a list of upper preferable values and lower preferable values, this is to be understood as specifically disclosing all ranges formed from any pair of any upper range limit or preferred value and any lower range limit or preferred value, regardless of whether ranges are separately disclosed. Where a range of numerical values is recited herein, unless otherwise stated, the range is intended to include the endpoints thereof, and all integers and fractions within the range. It is not intended that the scope of the invention be limited to the specific values recited when defining a range.

The present invention is directed to an aqueous personal care composition comprising a conditioner and polymers functionalized with amino groups and having the following structure (I) that provided improved conditioning to hair when incorporated into a model shampoo formulation containing detersive surfactants, and a foaming agent. The amino group is pendant to the polymer backbone (Structure I): 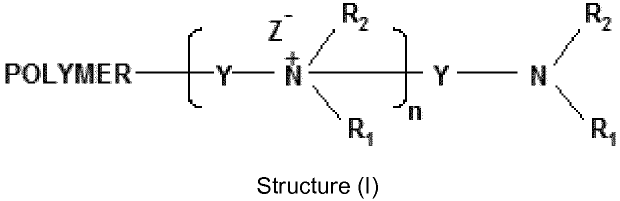
wherein the polymer comprises a polygalactomannan having a plurality of hydroxyl groups, wherein at least one of the hydroxyl groups reacts to form an oxygen bond with Y of the amino-containing group, and wherein the polygalactomannan has silanol (-Si-OH) or silanolate (-Si-O-Na+) groups; wherein:
Y is a bivalent alkylene moiety or substituted bivalent alkylene moiety;
R₁ and R₂ are hydrogen, C₁-C₂₀ alkyl, C₆-C₂₀ aryl or C₇-C₂₀ alkyl(aryl) groups and are the same or different;
n is an integer between 1 and 10, preferably between 1 and 5; and
Z⁻ is a counteranion, and
wherein the conditioner a silicone.

The counteranions of use in the present invention may be a simple anion such as for example Cl⁻, Fl⁻, Br⁻ or S²⁻ an oxoanion, such as for example NO3⁻, NO²⁻, PO₄³⁻ or SO₄²⁻, or an anion from an organic acid such as for example C₂H₃O₂⁻ or HCO₂⁻.

Alternatively, the amino group can be part of the polymer backbone (Structure II): 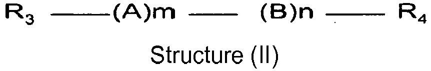 where A = the residue of a monomer or a group of monomers devoid of an amino group and B is an aminating monomer or a group of aminating monomers having the Structure (III), m is an integer between 0 and 100 and n is an integer between 1 and 100; R₃ and R₄= H or C₁-C₂₅ hydrophobic groups 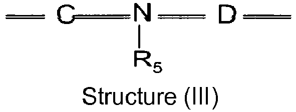 where -C-N (R₅)-D- is the residue of the aminating monomer(s) and C and D are hydrophilic or hydrophobic functionalized moieties and R₅ is H or C₁-C₂₅ hydrophobic groups.

The polymers functionalized with amino groups can be water-soluble, water-swellable or water-dispersible.

The present invention also discloses processes to manufacture various polymers functionalized with amino groups.

One embodiment of the present invention is directed to aqueous personal care compositions containing about 0.01% to about 10 wt% of one or more conditioners selected from the group consisting of cationic surfactants, cationic polymers, nucleic acids, lipids, silicones, hydrocarbon oil, fatty esters and combinations thereof.

The conditioners of use in the present invention comprise silicones. Preferably, the silicone may be selected from the group consisting of polyorganosiloxanes, polyorganosiloxane polyether copolyols, amodimethicones, and cationic polydimethylsiloxane materials.

The polyorganosiloxane conditioner can be in various forms such as polymers, oligomers, oils, waxes, resins, or gums.

The silicone may contain a functional group selected from the group consisting of amino, ammonium, hydroxyl, epoxy and polyalkylene glycols.

Another aspect of the of the present invention is directed to a method of treating keratinous substrates comprising the steps of applying the personal care composition to keratinous substrates and washing keratinous substrates with water.

### DETAILED DESCRIPTION OF THE INVENTION

In one embodiment of the present invention, the polymers functionalized with amino groups provide improved conditioning benefits to keratinous substrates and yet are substantially removable from the substrate surface when subsequently washed with a cleansing agent.

The concept of providing improved conditioning benefits to keratinous substrates which are substantially removable from the substrate surface when subsequently washed is demonstrated using polymers functionalized with amino groups, such as amino substituted guars and cellulosics. However, other polymers functionalized with amino groups would also provide similar conditioning benefits. The amino group can be primary, secondary or tertiary. If the amino group is secondary or tertiary in nature, the nitrogen atom of the amino group can be substituted with alkyl groups. The alkyl group would carry C1-C20 carbon atoms or a mixture thereof.

The polymers functionalized with amino groups of the present invention have unique structural features in that during the amination process quaternization of the tertiary amino groups occurs leading to the formation of amino substituents of the following structure: 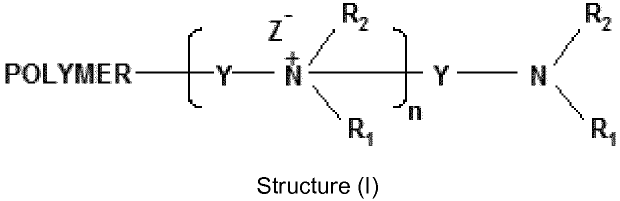 wherein the polymer comprises a polygalactomannan having a plurality of hydroxyl groups, wherein at least one of the hydroxyl groups reacts to form an oxygen bond with Y of the amino-containing group, and wherein the polygalactomannan has silanol (-Si-OH) or silanolate (-Si-O-Na+) groups; wherein:
Y is a bivalent alkylene moiety or substituted bivalent alkylene moiety;
R₁ and R₂ are the same or different and are hydrogen, C₁-C₂₀ alkyl, C₆-C₂₀ aryl or C₇-C₂₀ alkyl(aryl) groups and ;
n is an integer between 1 and 10, preferably between 1 and 5; and
Z⁻ is a counteranion, such as simple anion, an oxoanion, or an anion from an organic acid, and wherein the conditioner a silicone.

The personal care composition of the present invention may further comprise at least one additional ingredient selected from the group consisting of surfactant, rheology modifier, foaming agent, emulsifying agent, colorant, fragrance, preservative, antimicrobial agent, bleaching agent, lubricating agent, viscosifying agent, slippery agent, opacifiers, and mixtures thereof. These additional ingredients include cationic deposition polymers, suspending agents or rheology modifiers, other surfactants, fatty esters, fatty alcohols, polyalkylene glycols, inorganic compounds, fragrance, colorant, biocides, zinc-based antibacterial/antifungal agents (zinc pyrithione), fluorinated compounds, propellants, mono- and poly-valent metal salts, vitamins, proteins, coloring agents, opacifiers, amino acids, alpha-hydroxy acids and other components known for use in hair care and personal care products.

The use level of these individual components may range from 0.001% to 12% by weight based on the total weight of the personal care compositions. Cationic deposition polymers are those that bear cationic groups and form a coacervate with anionic surfactants present in the shampoo formulations. By forming a coacervate, they aid in depositing various active ingredients, such as silicone, zinc pyrithione, vitamins, proteins, fragrance, etc. typically present in personal care products. Cationic polymers used in personal care industry are described under the name "polyquatemium" which is the International Nomenclature for Cosmetic Ingredients (INCI) designation for cationic polymers. INCI has approved at least 37 different polymers under the "polyquatemium" designation. Different polymers are distinguished by the numerical value that follows the word "polyquatemium". The polymers of use in the present invention may comprise cationic polymers and may be selected from the group consisting of cationic hydroxyethylcellulose, hydrophobically modified cationic hydroxyethylcellulose, cationic starches, cationic polygalactomannans, cationic hydroxyalkylated polygalactomannans, and cationic diallyldimethylammonium chloride. Preferably, the cationic polymers of use in the present invention comprise cationic polygalactomannan.

The polymers functionalized with amino groups, preferably comprise a natural polymer selected from the group consisting of cellulose, starch, chitosan, xanthan, dextran, gellan, wellan gum, scleroglucan, alginic acid and its salts, carageenans, poly(β-hydroxyalkanote) and their copolymers, pectin and protein. The polymer functionalized with amino groups comprises aminated polygalactomannans. Polygalactomannans are natural polymers composed of mannose and galactose residues and are present in the seeds of leguminous plants, such as carob, guar, locust bean, tara and cassia tora. Their linear backbone is composed of 1 →6-linked β-D-mannose units with randomly recurring α-D-galactose side groups. The galactose unit is attached through its number 1 carbon atom to the number 6 carbon atom of the mannose unit through a glycosidic linkage. The average molar ratio of mannose to galactose in a given polygalactomannan varies depending on its source. The average mannose to galactose molar ratios in various polygalactomannans are shown below:

| **Polygalactomannan** | **Mannose :galactose (molar ratio)** |
|---|---|
| Fenugreek gum | 1:1 |
| Guar gum | ∼2:1 |
| Tara gum | 3:1 |
| Locust bean gum | 4:1 |
| Cassia gum | 5:1 |

The higher the galactose content of a polygalactomannan, the higher the solubility in water. With increase in galactose to mannose molar ratio, the inter-chain interactions loosen and consequently the rheological properties differ.

Polyglucomannans, such as konjac, are natural polysaccharides obtained from seeds or roots of certain plants. The structure is a random arrangement of 1 →4-linked β-D-glucose and β-D-mannose. The glucose to mannose molar ratio depends on the source and ranges from 1:1 in Iris bulbs to 1:5 in certain gymnosperms. In addition, they have a slightly branched structure (every 50-60 sugar units) via the carbon 3 of the sugars of the main chain and contain about one acetyl group per 19 sugar residues. Their molecular weights range from about 200,000 to about 2,000,000 Daltons depending on the source.

Preferably, the polymers functionalized with amino groups of the present invention are based on semisynthetic or completely synthetic polymers. They can be water-soluble, -swellable or -insoluble.

For further details on natural polymers, please see "Encyclopedia of Polymer Science, Vol. 11, Interscience Publishers, 1969, p. 396". The natural polymers can be directly functionalized with the amino groups. If the starting natural polymer is a polysaccharide, it can be modified with functional groups to affect its water-solubility. Incorporation of suitable functional groups into natural polymers to affect its water-solubility is known in the art. These include incorporation of various anionic groups, cationic groups and nonionic groups. Examples of anionic groups include but not limited to are carboxyalkyl, sulfonate, sulfate, phosphonate and silanolate. The polyglactomannan has a silanol (-Si-OH) or silanolate (-Si-O Na<+>) groups. Natural polymers having silanol (-Si-OH) or silanolate (-Si-O-Na⁺) groups may be produced by the process described in US Patent No. 4,992,538. Examples of cationic groups include phosphonium, sulfonium, and quaternary ammonium groups. Examples of nonionic groups include but are not limited to hydroxyalkyl and short chain alkyl groups. The degree of substitution (DS) with these modifying groups would be 0.001 to 2.9, preferably between 0.002 and 2.5 and most preferably between 0.1 and 2. Alternatively, the molar substitution of the polymer may describe the relative amount of amino substitution found on the polymer which may be stated in terms of MS which is the average number of moles of the amino group per mole of polymeric unit. Hydrophobic moieties can also be incorporated into the polymer to tailor the surface activity and rheological properties of the aminated polymer. The hydrophobe can be separately attached to the polymer or it can be attached to the nitrogen center of the amino group. Aminating reagents with hydrophobe(s) attached to the nitrogen center can also be used (see below) to confer hydrophobicity to the polymer.

Preferably, the above natural polymers or modified natural polymers can be reacted with appropriate aminating reagents capable of reacting with the functional groups such as - OH, =N-H, -SH and -COOH. Aminating reagents capable of reacting with polymers bearing active hydrogens include halogenoalkylamines or their salts, epoxyalkylamines, aziridines, isocyanatoamines or substituted isocyanatoamines. If needed, one or more of the =N-H bonds present on the nitrogen center of the amino group can be made temporarily unreactive by blocking them with suitable protecting agents, such as t-butyloxycarbonyl or trimethylsilylethyloxycarbonyl or (dimethyl)-t-butylsilyl group.

The amino groups in the polymers of the present invention are covalently bonded to the polymer through appropriate chemical linkages so that they remain attached to the polymer over a pH range of 3-11. There could be a spacer between the nitrogen center of the amino group and the point of attachment to the polymer. Halogenoalkylamines are preferred aminating agents. Examples of halogenoalkylamines include N,N-dimethylaminoethyl chloride, N,N-diethylaminoethyl chloride, N, N-dimethylaminopropyl chloride, N- methyl-N-ethylaminopropyl chloride, N-(2-chloroethyl)morpholine, N-(2-chloroethyl)dibenzylamine, N-ethyl-N-phenyl-aminoethyl chloride, 1-(3-chlorophenyl)-4-(3-chloropropyl)piperazine, 3-chloropropylamine. Some of these halogenoalkylamines are commercially available as their hydrochloride salts, such as N,N-dimethylaminoethyl chloride hydrochloride, N,N-diethylaminoethyl chloride hydrochloride and 3-chloropropylamine hydrochloride.

The aminated groups can be incorporated into the polymer backbone by reacting the aminating reagent with the polymer in the presence of a base. Examples of bases that can be used include alkali metal hydroxides, alkali metal carbonates or bicarbonates and tertiary amines. Preferred base is sodium hydroxide. If hydrohalide salts of the aminating reagent are used, the molar ratio of the hydrohalide reagent to the base should be at least 1:2, preferably 1:2.3 and most preferably 1:2.1.

The amination of the polymer can be carried out in the presence or absence of an inert organic solvent at a suitable temperature for a suitable length of time in the presence of water. The preferred mode of carrying out the amination is in the absence of an organic solvent as organic solvents are likely to be released to the environment during their processing causing environmental pollution. If needed, a phase transfer catalyst can be used to facilitate amination of the polymer.

Depending on the nature of the polymer to be aminated, processing aids, such as boron-containing compounds, polyvalent metal salts and other temporarily cross-linking agents can be used at appropriate stages of aminating the polymer. For polymers, bearing cis-diol functionality, borax (sodium tetraborate decahydrate, Na₂B₄O₇.10H₂O) can be used to cross-link the polymer chains to prevent dissolution of the polymer in the presence of water at elevated temperatures. Examples of natural polymers bearing cis-diol functionality are polygalactomannans that have pendant galactose groups. Polymers bearing cis-diol groups react with alkaline borax solution to form cross-linked structures that are water-insoluble at pH >7. Therefore, to aid in processing, that is to prevent excessive hydration and solubility of the polymer in an aqueous environment during amination and subsequent purification, such cis-diol bearing polysaccharides that are water-soluble or -swellable can be cross-linked with alkaline borax solution. The amount of borax used in the process should be such that the polymer is cross-linked enough with the borax to prevent its dissolution in water during amination and subsequent purification with water and yet majority of the reactive groups remain available for amination. In so doing, the polymer could be uniformly aminated.

For aminating seed-based polygalactomannans, such as fenugreek gum, guar gum, tara gum, cassia gum, locust bean gum and carob gum, the seed can be used "as is" or after grinding it. The preferred material would be the endosperm of these seeds that are enriched with polygalactomannans. For aminating guar gum polygalactomannans, endosperms from guar seeds, commonly referred to as "guar splits," can be used. For practicing the present invention, guar splits, free of hulls and germs are the most preferred source of guar gum polygalactomannans. The guar splits can be used "as is" or in the form of a powder having sufficient surface area. Guar splits are commercially available.

The modification and functionalization of polymers functionalized with amino groups can be carried out sequentially or simultaneously.

Other approach of temporarily cross-linking natural polymers or modified natural polymers involves the reaction of the polymer with glyoxal under acidic environment (glyoxalation), typically at pH between 3 and 5. Since hemiacetal linkages formed by glyoxalation are unstable at pH >7 and amination is done typically at pH >7, it is preferred that the glyoxalation of the polymer is carried following amination of the polymer. Glyoxalation of the polymers functionalized with amino groups can be carried out before or after purification of the polymer.

Another approach to prepare polymers functionalized with amino groups involves the reaction of a polymer bearing epoxy groups with an amine bearing an -N-H group. Examples of epoxy alkylamine include 3-diethylamino-1, 2-epoxypropane and 3-dimethylamino-1, 2-epoxypropane. For amination using epoxy amines, only catalytic amount of a base is needed and the amount of the base is generally about 0.3 to about 10% based on the weight of the polymer to be aminated.

Yet another approach of making polymers functionalized with amino groups is to first incorporate an epoxy group into the polymer first by functionalizing the polymer with vinyl groups followed by epoxidation of the vinyl groups. The preparation of polymers functionalized with amino groups according to this approach is schematically shown below using a polymer bearing -OH groups.

Polymer-OH + X-CH2CH=CH2 --------> Polymer-O-CH2CH=CH2

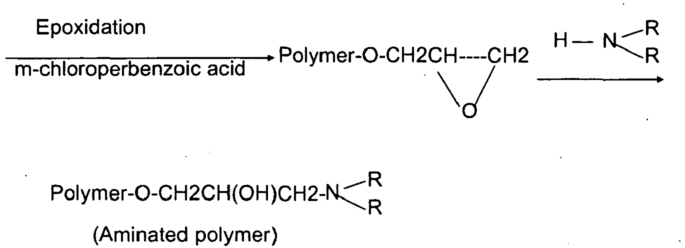

The molecular weight of natural polymers of use in the present invention range from about 100,000 to about 2,000,000 Daltons. Depending on the application needs, the molecular weight of the polymer can be further increased or reduced by either chemical cross-linking or chain scission respectively using a number of methods known in the art to tailor molecular weight of natural polymers in general and polysaccharides in particular. Chemical methods of molecular reduction for polysaccharides involves chain scission using acids, alkalies, oxygen, ozone, hypochlorite, hydrogen peroxide, organic hydroperoxides such as for example butyl hydroperoxide in conjunction with a metal catalyst, and appropriate enzymes. For enzymatically degrading polysaccharides, appropriate enzyme can be used. For examples, cellulose, starch and polygalactomannan based polymers functionalized with amino groups can be hydrolyzed with cellulose, amylase and mannase (L. R. S. Moreira and E. X. F. Filho, Appl. Microbiol. Biotechnol., 79, 165, 2008) respectively. A chemo-enzymatic method, that is, a combination of chemical and enzymatic method can also be used to lower the molecular weight of aminated polysaccharides of the present invention. For aminated proteins, proteases can be used to bring about their molecular degradation. The tailoring of molecular weight can be done in a high solids process or in solution using water or a mixture of water and organic solvents before or after the polymers are functionalized with amino groups.

The polygalactomannans of the present invention, may contain proteins and/or aminated proteins. This is due to the fact that polygalactomannans may contain proteinaceous materials and other non-polygalactomannan polysaccharides as impurities. These proteinaceous materials and non-polygalactomannan polysaccharides can chemically react with the aminating reagent or other functionalizing agents to form the corresponding amine functionalized derivatives. These derivatives may not be completely removed during the purification of the aminated polygalactomannans. Typically, the nitrogen content of the aminated polygalactomannans contributed by protein residues ranges from about 0.01 % to about 1%.

Preferably, the polymers functionalized with amino groups are produced from cellulose derivatives, more preferably cellulose ethers, aminated cellulose ethers can be made using various cellulose ether polymers. These include, but not limited to, hydroxyethylcellulose, hydroxypropylcellulose, carboxymethylcellulose, carboxymethyl hydroxyethylcellulose, methylcellulose, methylhydroxyalkylcelluloses, hydroxyethylhydroxy propylcellulose, hydrophobically modified hydroxyethylcellulose. Another commercially available cellulose ether that can be used as a base polymer to prepare the aminated polymer is ethyl hydroxyethylcellulose. Processes for making amino derivatives from carboxyl-containing polysaccharides including carboxymethylcellulose are disclosed in U. S. Patent No. 4,963,664. U. S. Patent No. 3,431, 254, describes the preparation of aminoalkylated hydroxypropylcellulose. Various anionic groups, cationic groups and nonionic groups may be incorporated into the cellulose derivatives. Examples of anionic groups include, but not limited to, carboxyalkyl, sulfonate, sulfate, phosphonate and silanolate. Of particular interest are cellulose derivatives having silanol (-Si-OH) or silanolate (-Si-O-Na⁺) groups. These cellulose derivatives having silanol (-Si-OH) or silanolate (-Si-O-Na⁺) groups may be produced by the process described in US Patent No. 4,992,538.

Preferably, Chitosan, made by deacetylation of chitin, bears -NH₂ groups and can be used to practice the present invention. It can be modified with other amino groups using appropriate aminating reagents, and the aminated chitosan can also be used to practice the present invention.

Preferably, the polymers functionalized with amino groups are produced from starch ethers, the resultant aminated starch ethers can also be used to practice the present invention. Preparation of diethylaminoethylated starch is known (E. A. EI-Alfy, S. H. Samaha and F. M. Tera, Starch, Volume 43, Issue 6, 235, 1991 ; Li-Ming Zhang and Dan-Qing Chen, Starch, Volume 53, Issue 7, 311, 2001). Other aminated starch derivatives can be made by post modifying starch ethers with appropriate aminating agents. These include hydroxyethyl starch, hydroxypropyl starch and hydrophobically modified starches. Naturally occurring starches are mixtures of two polysaccharides; a) amylose, an essentially linear polymer of α-D-glucopyranose connected by 1→4 α-linkages and b) amylopectin, a highly branched polymer of α-D-glucopyranose. Amylopectin is comprised of short linear chains of amylose (degree of polymerization ranging from 12-50 α-D-glucopyranose units) and bears branches composed of amylose chains of various lengths that are connected to the amylose backbone through 1→6 α-linkages. The relative amount of these two types of polymers in a starch sample depends on the source and determines the functional properties of the starch or starch derivatives.

### Polymers functionalized with amino groups based upon synthetic polymers

A wide variety of synthetic water-soluble polymers useful in the present invention are commercially available. These synthetic polymers are selected from the group consisting of polyalkylene glycols and their copolymers, polyvinyl alcohols, polyvinyl amine, homo and copolymers of acrylic acid, homo and copolymers of acrylic and methacrylic acid, homo and copolymers of acrylamide, polyethyleneimines, polyvinylpyrrolidone, poly(ether-polyurethanes), poly(ether-polyols), poly(acetal-polyethers) and their copolymers. These preformed polymers can be post modified with appropriate
aminating reagents under suitable reaction conditions either in solution, in a slurry form, molten state or in a high solids process in an aqueous environment. The preparation of novel aminated poly(acetal-polyethers) is disclosed in U.S. Patent No. 6,162,877.

These preformed polymers can be modified with appropriate aminating reagents under suitable reaction conditions described under "Polymers functionalized with amino groups based upon natural polymers" hereinabove. Particularly, synthetic polymers bearing -OH, -SH and =N-H can be post modified with aminating reagents using appropriate reaction conditions disclosed under "Polymers functionalized with amino groups based upon natural polymers" hereinabove. Examples of such polymers include polyalkylene glycols, poly(vinyl alcohol-co-vinyl acetate), and poly(vinyl alcohol-co-vinyl amine).

Alternatively, synthetic polymers functionalized with amino groups can be made by copolymerizing non-aminated polymerizable monomers with aminated monomers. Examples of aminated monomers include 2-(N,N-diethylaminoethyl) acrylate, 2-(N,N-diethylaminoethyl) methacrylate, 2-(diisopropylaminoethyl) methacrylate, 3-(dimethylaminopentyl) acrylate, N-[3-(N,N-dimethylaminopropyl)] acrylamide and N-[3-(N,N-dimethylaminoethyl)] acrylamide.

The molecular weight of the synthetic polymers functionalized with amino groups ranges from about 500 to about 30,000,000 Daltons. The mole% of the aminated monomer in the polymer is from about 5% to about 90%. Preferably, the mole% of the aminated monomer is from about 10% to about 80%, and still more preferably from about 20% to about 70%. Multiple aminated monomers and non-aminated monomers can be used to prepare an aminated polymer of the present invention.

Another aspect of the present invention is that the synthetic polymers functionalized with amino groups of the present invention contain cationic groups in addition to amino groups. These cationic groups impart positive charge to the synthetic polymers functionalized with amino groups at a pH of about 2 to about 13. Examples of such cationic groups include -2-hydroxypropropyltrialkyl ammonium halide (-CH₂-CH(OH)CH₂-N⁺(R₃) X⁻; R₃ = an alkyl group having the same or different number of carbon atoms; X = chloride, bromide or iodide), sulfonium and phosphonium groups. The cationic charge density arising from these cationic groups should be less than about 1 meq/g.

The cationic reagent that can be used to incorporate cationic groups into the polymer can have either of the following structures: 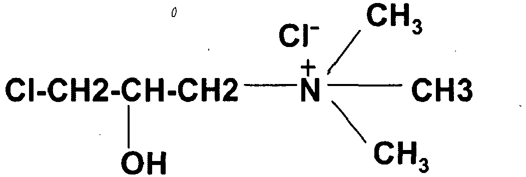 or

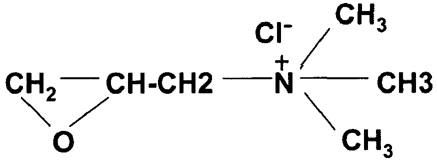

To incorporate cationic functionality into the polymer backbone, a cationic reagent can be reacted first with the polymer functionalized with amino groups. Alternatively, the polymer can be first functionalized with the cationic groups and then the resulting cationic polymer can further be functionalized with amino groups. Yet another method to incorporate cationic groups would be to react the polymer simultaneously with the amine functionalizing reagent and the cationic reagent. The relative amount of cationic functionalization and amino functionalization can be tailored by adjusting the amounts of the cationizing agent and the amino functionalizing agent with respect to the weight of the polymer.

Depending on the molecular weight of the polymers functionalized with amino groups, they can be made by solution, suspension, precipitation or emulsion polymerization processes.

The conditioning benefit of the synthetic polymers functionalized with amino groups is achieved by incorporating them into various personal care formulations that contain other active and inactive ingredients. For hair care applications, they may be used in shampoo formulations. A model shampoo formulation contains detersive surfactants, foaming agent, a silicone conditioner, and sodium chloride.

Incorporation of synthetic polymers functionalized with amino groups provided improved conditioning as judged by the lower energy needed to comb the hair treated with the shampoo containing sodium laureth sulfate (with 1-3 moles of ethylene oxide), sodium lauryl sulfate, cocamidopropyl betaine and silicone. They also provided significant enhancement in silicone deposition-a functionality that is highly desirable by shampoo manufacturers to deliver superior conditioning using less silicone in the shampoo formulations and enhance the stability of shampoo (prevention of phase separation) on long term storage.

Besides containing synthetic polymers functionalized with amino groups of the present invention, the personal care compositions may contain cationic polymers and rheology modifiers. The preferred cationic polymers could be synthetic or natural polymers with a molecular weight from about 1,000 to about 10,000,000 Daltons and carrying a positive charge density of about 0.3 meq/g (milliequivalent per gram) to about 10 meq/g.

The amino groups of the aminated polymers of the present invention can be partially or fully converted to cationic groups by reacting the aminated polymer with a halogenoalkane or an epoxyalkane. If a halogenoalkanoic acid is used, for example, chloroacetic acid or its alkali metal salts, a zwitterionic polymer would be formed. Such polymers could also be used in various personal care products including shampoos.

To control the rheology of the personal care products, additional water-soluble or -swellable polymers can be incorporated. These include but not limited to are carboxylic acid polymers, crosslinked polyacrylate polymers, polyacrylamides and mixtures thereof.

### STANDARD TESTING PROCEDURES

### Molecular Weight

Weight average molecular weights were determined using aqueous size exclusion chromatography.

### Performance of Personal Care Compositions

Wet and dry hair combability measurements are typical test methods used to measure conditioning performance in shampoo and conditioner applications.

Silicone deposition onto hair tresses from shampoos can be measured by several techniques. One technique used for quantifying silicone deposition onto hair is described below.

### Silicone deposition measurement

Each 2-5 gram hair tress sample was weighed to the nearest mg, after removal of sample holder, and placed into clean 8 oz jars with approximately 150 ml of methylene chloride. The samples were shaken for 1.5 hours at room temperature. The methylene chloride supernatant was filtered using Whatman # 41 filter paper and quantitatively transferred to clean 8 oz jars and evaporated to dryness with mild heat and a nitrogen sparge. Each sample was then dissolved into 2 ml of chloroform-d and quantitatively transferred to a 5-ml volumetric flask. Three chloroform-d rinses were used to transfer each sample to the 5-ml volumetric flask. All flasks were diluted to the mark with solvent and inverted. Each sample was examined in a Nicolet Magna 550 FT-IR with 150 co-added scans at 4 cm-1 resolution and 0.4747 velocity using a 0.1 cm-fixed path salt cell. A chloroform-d reference spectrum was used to subtract out the solvent bands (diff=1.0). The silicone level was determined by measuring the peak height of the Si-CH₃ stretch at 1260 cm-1 (baseline 1286 and 1227 cm-1) followed by conversion to mg/ml of silicone using a low level calibration curve extending from 10 - 300 parts per million (ppm). Each sample was corrected for dilution volume and sample weight. All values are reported to the nearest ppm.

Wet/Dry Comb Performance Measurement- Virgin Medium Brown European Hair Conditions:
Measured at constant temperature and humidity (22.2°C) and 50 % relative humidity).

### Equipment:

Instron 1122 (907 g (2-lb.) Load cell, 500-gram range used)

### Prewash procedure:

Each tress was washed twice with sodium lauryl sulfate (SLS) or other anionic surfactants, e.g., sodium lauryl ether sulfate (SLES) using 0.1g-5g surfactant/gram tress, washing twice with water and then air drying overnight at 22.2°C (73°F) and 50% relative humidity. The twice washed tress was hand combed 5-times with large teeth comb and 5-times with small teeth comb (10x total).

The following combing protocol was used for virgin hair. Two to three tresses were used, and the average reported from the two to three tresses combed 8 times per tress, with more precombing of the tresses prior to measurement as described above.

### Shampoo procedure

1. Each tress was shampooed twice with 0.1-0.5g shampoo per 1 gram tress (each tress weighed 3.0 g).
2. Each shampooed tress was hand combed twice with a large teeth comb.
3. The hand combed tress was loaded onto an Instron instrument and the crosshead was lowered to bottom stop. The tress was combed twice with small teeth comb and placed into double-combs.

The Instron was run under standard conditions.

After the test was run, the tress was sprayed with deionized water to keep it moist. Using a paper towel, excess liquid was wiped off the double-combs.

The crosshead was returned to bottom stop and tress replaced onto double-combs.

The tress was rerun under standard conditions. A total of eight tests were run on each tress.
4. After the eight tests were finished, the tress was hung up overnight.
5. The next day, each tress was dry combed and tested eight times.
6. The wet comb energy was averaged for 16 Instron runs and results reported with standard deviation.
7. The dry comb energy was averaged for 16 Instron runs and results reported with standard deviation.

### Viscosity

The viscosity of the compositions was measured using a Brookfield LVT viscometer with a spindle # 4, at 30 rpm and at 25°C.

The following examples will serve to illustrate the invention, parts and percentages being by weight unless otherwise indicated.

### EXAMPLE 1

### Amination of guar with diethylaminoethyl chloride hydrochloride (DEAECl.HCl)

To a high solids reactor, equipped with ribbons to facilitate mixing of solids or semisolid materials, was charged water (200 g), borax (2.6 g) and N,N-diethylaminoethyl chloride hydrochloride (100 g). After sealing the reactor, the contents of the reactor were heated to 80°C. Then the reactor was opened and guar splits (200 g; "as is"; moisture = 8%) were added under gentle agitation at 30 rpm. The reactor was sealed again, and the inside of the reactor was made oxygen-free and heated at 80°C for 30 minutes under constant agitation. Then the reactor contents were cooled to 65°C over a period of 15 minutes. After this, the reactor was opened and sodium hydroxide (49 g) was added to the hydrated guar. Following the addition of sodium hydroxide, the reactor was sealed and the inside of the reactor was made oxygen-free. The resulting reaction mixture was heated at 65°C for 3 h, cooled to 35°C and opened. The reaction mixture was collected, washed four times with water and the purified polymer was dehydrated with acetone. The acetone dehydrated polymer was allowed to air dry for 24 h.

The volatile content of the air-dried diethylaminoethylated guar (DEAE-guar) was 19%. The 1.6% solution Brookfield viscosity at pH ∼6 was 15,300 cps at 25°C at 30 rpm using spindle # 4. The solution was slightly hazy.

The DEAE molar substitution of the DEAE-guar was 0.34 as measured by ¹H NMR spectroscopy. In addition to the DEAE group, the aminated guar was likely to contain groups of the following general structure.

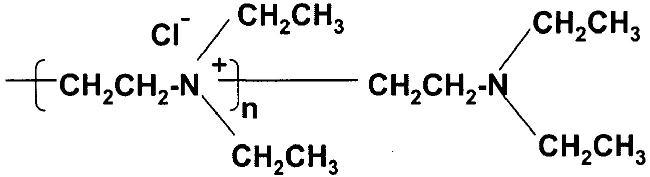

### EXAMPLE 2

Amination of guar with dimethylaminoethyl chloride hydrochloride in the presence of NaOH.

Example 1 was repeated using dimethylaminoethyl chloride hydrochloride. The amounts of various reagents used were:
a) Guar split - 200 g
b) Water - 200 g
c) N,N-Dimethylaminoethyl chloride hydrochloride - 84 g
d) Borax - 2.8 g
e) Sodium hydroxide - 49 g

The volatile content of the air-dried dimethylaminoethylated guar (DMAE-guar) was 9.8%. The 1.6% solution Brookfield viscosity at pH ∼6 was 5650 cps at 25°C at 30 rpm using a spindle # 4. The solution was hazy.

The DMAE molar substitution of the DMAE guar was 0.108 measured by ¹H NMR spectroscopy.

### EXAMPLE 3

### Modification of cationic guar with DEAE groups.

Cationic guar (cationic degree of substitution = 0.18) (400 g; moisture ∼50 wt%), made by reacting guar split with 65% solution of 3-chloro-2-hydroxypropyltrimethylammonium chloride (Dow Quat 188 cationic reagent, available from the Dow Chemical Company) in the presence of sodium hydroxide was charged to the high solids reactor. Then under gentle agitation were added sodium hydroxide (25 g) and N,N-diethylaminoethyl chloride hydrochloride (52 g). After sealing the reactor and making the inside of the reactor oxygen-free, the resulting reaction mixture was heated at 60°C for 4 h. The reactor was then cooled to 40°C, opened and the reaction mixture was collected. The crude reaction mixture was purified by washing with 80:20 methanol/water and acetone. The acetone washed material was dried in air for 24h.

The volatile content of the air-dried diethylaminoethylated cationic guar (DEAE cationic guar) was 15.3%. The 1.6% solution Brookfield viscosity at pH ∼6 was 260 cps at 25°C at 30 rpm using spindle # 4. The solution was almost clear. The weight average molecular weight of the DEAE cationic guar was about 600,000 Daltons.

A1% solution of the DEAE cationic guar was dialyzed in a water bath for 10 days using Spectrapor® cellulose acetate membrane (cutoff MW ∼ 5000) (available from Spectrum Laboratories Inc.) to remove low molecular weight non-guar impurities. Analysis of the dialyzed sample by ¹H NMR showed that the DEAE cationic guar had a cationic degree of substitution of 0.175 and DEAE molar substitution was 0.125.

### EXAMPLE 4

### Reaction of guar with DEAECl·HCl and 3-chloro-2-hydroxypropyltrimethyl Ammonium chloride

### Preparation of diethylaminoethylated cationic guar

First, a DEAE-guar was made according to Example 1 using the following reagents.
a) Guar split - 200 g
b) Water - 200 g
c) N,N-Diethylaminoethyl chloride hydrochloride - 100 g
d) Borax - 2.6 g
e) Sodium hydroxide - 49 g

To this DEAE guar at 35°C were added *in situ* 65% aqueous solution of 3-chloro-2-hydroxypropyltrimethylammonium chloride (Dow Quat 188 cationic reagent, available from the Dow Chemical Company) (84.5 g) and sodium hydroxide (9 g). After sealing the reactor, the reactor content was made oxygen free, heated at 60°C for 1.5 h and cooled to 35°C.

The reaction mixture was collected, washed four times with water and the purified polymer was dehydrated with acetone. The acetone dehydrated polymer was allowed to air dry for 24 h.

The volatile content of the air-dried diethylaminoethylated cationic guar (DEAE cationic guar) was 15.2%. The 1.6% solution Brookfield viscosity at pH ∼6 was 3245 cps at 25°C at 30 rpm using a # 4 spindle. The DEAE cationic guar solution was hazy. The weight average molecular weight of the DEAE cationic guar was about 1,330,000 Daltons.

Analysis of the sample by ¹H NMR showed that DEAE cationic guar had a cationic degree of substitution of 0.16 and a DEAE molar substitution of 0.33. The fraction of the 3-chloro-2-hydroxypropyltrimethylammonium chloride grafted onto the nitrogen center of the diethylaminoethyl group was 15 %.

### EXAMPLE 5

### Preparation of (chlorophenyl)propylpiperazinylated hydrophobically modified hydroxyethylcellulose (CPPPZ-HMHEC)

Natrosol® Plus hydrophobically modified hydroxyethylcellulose (HMHEC) (Grade 330) (100 g) (available from Hercules Incorporated, Wilmington, Delaware) was slurried in a mixture of t-butyl alcohol (450 g) and sodium hydroxide solution (6 g of NaOH dissolved in 30 g water) and alkalized for 30 minutes in a nitrogen atmosphere. To this alkalized slurry was added a dispersion of 1-(3-chloropropyl)-4-(3-chlorophenyl) piperizine chloride hydrochloride, Cl(CH₂)₃-N(CH₂)₂-N-C₄H₄Cl, (2 g) in water (30 g).

The resulting reaction mixture was heated at 95°C for 4 h. Then the reaction mixture was cooled to room temperature, neutralized with 36% hydrochloric acid (12.6 g) and filtered. The residue was washed three times with 80:20 acetone/water mixture, and finally dehydrated with acetone. The dehydrated polymer was dried in a fluid bed dryer at 50°C for 0.5h.

Analytical data after dialysis: Moisture - 2.06%; Ash (Na₂SO₄) - 0.55%; Nitrogen - 0.132%; CPPPZ degree of substitution = 0.016.

### EXAMPLE 6

### Preparation of dibezylaminoethylated hydrophobically modified hydroxyethylcellulose (DBAE-HMHEC)

Example 5 was repeated using 4g of N-(2-chloroethyl)dibenzylamine hydrochloride as the aminating reagent.

Analytical data after dialysis: Moisture - 5.86%; Nitrogen - 0.088%; CPPPZ degree of substitution = 0.016; dibenzylaminoethyl degree of substitution - 0.022.

### EXAMPLE 7

### Preparation of N-ethyl-N-phenyl-aminoethylated hydrophobically modified hydroxyethylcellulose (EPAE-HMHEC)

Example 5 was repeated using N-(2-chloroethyl)-N-ethylamine as the aminating reagent. The reagents used were:
a) Natrosol® Plus HMHEC (Grade 330) - 100 g
b) t-Butyl alcohol - 450 g
c) Sodium hydroxide - 5 g
d) Water - 30 g
f) N-Ethyl-N-phenylaminoethyl chloride - 2.48 g
g) Hydrochloric acid -936%) - 11.3 g

Analytical data after dialysis: Moisture - 2.88%; Ash (Na₂SO₄) - 0.72%; Nitrogen - 0.053%; N-ethyl-N-phenylaminoethyl degree of substitution - 0.012 022.

### EXAMPLE 8

### Preparation of diethylaminoethylated hydrophobically modified hydroxyethylcellulose (DEAE-HMHEC)

Example 5 was repeated using N,N-diethylaminoethyl chloride hydrochloride as the aminating reagent. The reagents used were:
a) Natrosol® Plus HMHEC (Grade 330) - 100 g
b) t-Butyl alcohol - 450 g
c) Sodium hydroxide - 6 g
d) Water - 30 g
e) N, N-Diethylaminoethyl chloride hydrochloride - 2 g
f) Water - 30 g
f) Hydrochloric acid -936%) - 11.3 g

Analytical data after dialysis: Moisture - 1.79%; Ash (Na₂SO₄) - 1.02%; Nitrogen - 0.026%; diethylaminoethyl degree of substitution - 0.006

### EXAMPLE 9

### Preparation of diethylaminoethylated hydroxypropylcellulose (DEAE-HPC)

To a mixture of t-butyl alcohol (160 g), n-heptane (720 g), water (50 g) and sodium hydroxide (26 g) in a Chemco reactor was added Extranier PHV cellulose (84 g; "as is" weight). The resulting cellulose slurry was mixed at room temperature for 1 h in a nitrogen atmosphere. Through the reactor port was added N, N-diethylaminoethyl chloride hydrochloride (40 g). The reactor was closed and propylene oxide (240 g) was added. The resulting reaction mixture was heated at 75°C for 2h, at 85°C for 1 h and at 95°C for 3 h in a nitrogen atmosphere. Then the reaction mixture was cooled to room temperature.

The reaction mixture was filtered under suction, and the residue washed with boiling water at pH ∼8.5. The wet filtered cake was dried in a fluid bed dryer at 90°C for 1 h.

Analysis: Hydroxypropyl molar substitution - 3.8; Nitrogen - 0.86%; diethylaminoethyl degree of substitution - 0.24; 1% solution Brookfield viscosity at 25°C at 30 rpm using a spindle # 4 - 440 cps.

### EXAMPLE 10

### Preparation of 2-morpholinoethyl hydroxypropylcellulose

Example 5 was repeated using 43 g of 4-(2-chloroethyl)morpholine hydrochloride.

Analysis: Hydroxypropyl molar substitution - 4.25; Nitrogen - 0.96%; 2-morpholinoethyl degree of substitution - 0.33; 1% solution Brookfield viscosity at 25°C at 30 rpm using a # 4 spindle - 285 cps.

### EXAMPLE 11

### Preparation of diethylaminoethylated polyethylene glycol

To a high solids reactor were charged polyethylene glycol (molecular weight -4000) (500 g), N,N-diethylaminoethyl chloride hydrochloride (50 g) and sodium hydroxide (30 g). The reactor was sealed and the inside of the reactor was made oxygen-free. The resulting reaction mixture was heated at 80°C for 2 h to obtain the diethylaminoethylated polyethylene glycol.

¹H NMR spectrum of the sample confirmed the grafting of diethylaminoethyl groups onto the chain termini of the polyethylene glycol.

### EXAMPLE 12

### Preparation of aminated polvacetal-polvether bearing tertiary nitrogen centers in the backbone

To a high solids reactor were charged polyethylene glycol (molecular weight -4000) (500 g), Rhodameen T-15 (Available from Rhodia) (C₁₆/C₁₈-amine containing about 15 moles of ethylene oxide) (61 g) and sodium hydroxide (20.5 g). The reactor was sealed and the inside of the reactor was made oxygen-free. The resulting reaction mixture was heated at 75°C for 1 h. Then dibromomethane (40 g) was added to the molten alkalized polyethylene glycol, and the resulting reaction mixture heated at 80°C for 2 h to form the aminated polyethylene glycol copolymer.

Analytical: Weight average molecular weight - 40,400; polydispersity - 2.12; C₁₆ content - 0.13%; C₁₈ content - 0.21 %.

The aminated polyethylene glycol copolymer was water-soluble.

¹H NMR spectrum of the sample confirmed the incorporation of Rhodameen T-15 into the copolymer backbone. It had the following structure: 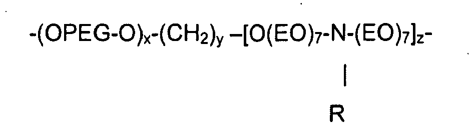 R = C₁₆ and C₁₈

### EXAMPLE 13

### Aminated polymers in model shampoo formulations

Three aminated polymers (DEAE-guars) produced in Example 12 were evaluated in model shampoo formulations containing sodium laureth sulfate (SLES; containing 2 moles of ethylene oxide) (12%), cocamidopropyl betaine (2%), sodium lauryl sulfate (SLS) (6%), sodium chloride (1%), and silicone (50% aqueous dispersion of Dimethicanol, available from Dow Corning) (1.5%). The amount of the aminated polymer used in the formulation was 0.2%. The pH of the shampoo was 5.4-5.8.

### Combing Test

The wet comb measurements were performed on an Instron instrument using virgin brown hair tresses that had been treated with the DEAE-guar as a conditioning polymer.

The work needed to comb a tress after treating with a conditioner was measured as grams of force times distance per gram of tress (gf-mm/g).

The work required to comb wet hair after treating the hair tresses with the conditioning compositions were measured. Also measured was the amount of silicone deposited onto hair in conjunction with various aminated polymers. The results are shown in Table 1.

**Table 1**

| **Wet comb energy and silicon deposition data for aminated guars** | | | |
|---|---|---|---|
| Designation | Conditioning polymer used in the shampoo formula | Wet comb work for virgin brown hair (gf-mm/g) | Silicon deposit on virgin brown hair (ppm) |
| | | | |
| Control experiment | No polymer used | 5485 | <10 |
| Commercial cationic guar | N-Hance 3270 cationic quar^{a} | 4266 | 40 |
| " | Jaguar C-17 cationic guar^{b} | 2588 | 96 |
| 12-A (Comparative) | Experimental cationic guar^{c} | 2901 | 86 |
| 12-B | DEAE Guar^{d} | 2483 | 230 |
| 12-C | DEAE Guar^{e} | 2441 | 579 |
| 12-D | Cationic modified DEAE guar^{f} | 2654 | 581 |

| | | | |
|---|---|---|---|
| ^{a}N-Hance 3270 cationic guar = A commercial cationic guar (cationic degree of substitution - 0.12 to 0.15), available from Hercules Incorporated, a subsidiary of Ashland, Inc. ^{b}Jaguar C-17 cationic guar = A commercial cationic guar (cationic degree of substitution - 0.2 to 0.25) available from Rhodia. ^{c}Experimental cationic guar; cationic degree of substitution (DS) - 0.34 ^{d}DEAE molar substitution (MS) = 0.28 ^{e}DEAE molar substitution (MS) = 0.34 ^{f}DEAE molar substitution (MS) = 0.33; cationic degree of substitution (DS) = 0.16 | | | |

As can be seen in Table 1, DEAE-guars as conditioning polymers significantly reduced wet comb work for virgin brown hair. The reduced comb work is consistent with the amount of silicone deposited on hair. The higher the amount of silicone deposited on the hair, the lower the wet comb work. The amount of silicone deposited on hair was measured as the amount of elemental silicon (Si) deposited on hair and is expressed in ppm (parts per million).

## Claims

1. An aqueous personal care composition comprising a conditioner and a polymer functionalized with an amino-containing group, wherein the amino-containing group is pendant and the polymer functionalized with the amino-containing group has the following structure: 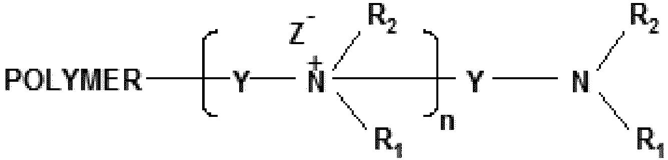 wherein the polymer comprises a polygalactomannan having a plurality of hydroxyl groups, wherein at least one of the hydroxyl groups reacts to form an oxygen bond with Y of the amino-containing group, and wherein the polygalactomannan has silanol (-Si-OH) or silanolate (-Si-O⁻ Na⁺) groups; wherein:
Y is a bivalent alkylene or substituted bivalent alkylene moiety;
R₁ and R₂ are the same or different and are hydrogen, C₁-C₂₀ alkyl, C₆-C₂₀ aryl or C₇-C₂₀ alkyl(aryl) group;
n is an integer between 1 and 10;
Z⁻ is a counteranion, and
wherein the conditioner comprises a silicone.

2. The aqueous personal care composition of claim 1, wherein the silicone is selected from the group consisting of polyorganosiloxanes, polyorganosiloxane polyether copolyols, amodimethicones, and cationic polydimethylsiloxane materials.

3. The aqueous personal care composition of claim 2, wherein the silicone contains a functional group selected from the group consisting of amino, ammonium, hydroxyl, epoxy and polyalkylene glycols.

4. The aqueous personal care composition of claim 1, wherein the aqueous personal care composition further comprises at least one additional ingredient selected from the group consisting of surfactant, rheology modifier, foaming agent, emulsifying agent, colorant, fragrance, preservative, antimicrobial agent, bleaching agent, lubricating agent, viscosifying agent, slippery agent, opacifier, salt and mixtures thereof.

5. The aqueous personal care composition of claim 1, wherein the polygalactomannan is selected from the group consisting of fenugreek gum, guar gum, tara gum, locust bean gum and cassia gum.

6. The aqueous personal care composition of claim 5, wherein the polygalactomannan comprises guar.

7. The aqueous personal care composition of claim 1, wherein Z⁻ comprises a simple anion, an oxoanion, or an anion from an organic acid.

8. The aqueous personal care composition of claim 1, wherein the composition further comprises a cationic polymer.

9. The aqueous personal care composition of claim 8, wherein the cationic polymer is selected from the group consisting of cationic hydroxyethylcellulose, hydrophobically modified cationic hydroxyethylcellulose, cationic starches, cationic polygalactomannans, cationic hydroxyalkylated polygalactomannans, and cationic diallyldimethylammonium chloride.

10. The aqueous personal care composition of claim 9, wherein the cationic polymer comprises cationic polygalactomannans.

11. The aqueous personal care composition of claim 10, wherein the cationic polygalactomannan comprises cationic guar.

12. The aqueous personal care composition of claim 1, wherein n is an integer between 1 and 5.

## Patentansprüche

1. Wässrige Körperpflegezusammensetzung, umfassend ein Konditionierungsmittel und ein Polymer, das mit einer aminohaltigen Gruppe funktionalisiert ist, wobei die aminohaltige Gruppe seitenständig ist und das mit der aminohaltigen Gruppe funktionalisierte Polymer die folgende Struktur aufweist: 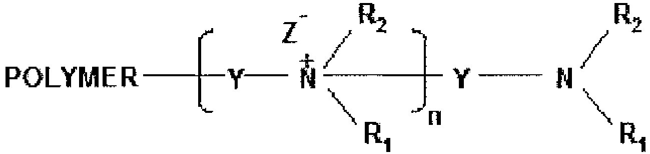 wobei das Polymer ein Polygalactomannan mit einer Vielzahl von Hydroxylgruppen umfasst, wobei mindestens eine der Hydroxylgruppen reagiert, um eine Sauerstoffbindung mit Y von der aminohaltigen Gruppe zu bilden, und wobei das Polygalactomannan Silanol-(-Si-OH) oder Silanolat- (-Si-O- Na⁺) -Gruppen aufweist; wobei:
Y ein zweiwertiger Alkylen- oder substituierter zweiwertiger Alkylenanteil ist;
R₁ und R₂ gleich oder unterschiedlich sind und Wasserstoff, C₁-C₂₀-Alkyl, C₆-C₂₀-Aryl oder C₇-C₂₀-Alkyl-(aryl) gruppe sind;
n eine ganze Zahl zwischen 1 und 10 ist;
Z⁻ ein Gegenanion ist, und
wobei das Konditionierungsmittel ein Silikon umfasst.

2. Wässrige Körperpflegezusammensetzung nach Anspruch 1, wobei das Silikon ausgewählt ist aus der Gruppe bestehend aus Polyorganosiloxanen, Polyorganosiloxan-Polyether-Copolyolen, Amodimethiconen und kationischen Polydimethylsiloxanmaterialien.

3. Wässrige Körperpflegezusammensetzung nach Anspruch 2, wobei das Silikon eine funktionale Gruppe ausgewählt aus der Gruppe bestehend aus Amino, Ammonium, Hydroxyl, Epoxy und Polyalkylenglykolen enthält.

4. Wässrige Körperpflegezusammensetzung nach Anspruch 1, wobei die wässrige Körperpflegezusammensetzung ferner mindestens einen zusätzlichen Bestandteil ausgewählt aus der Gruppe bestehend aus Tensid, Rheologiemodifizierungsmittel, Schäumungsmittel, Emulgator, Färbungsmittel, Duftstoff, Konservierungsmittel, antimikrobiellem Mittel, Bleichmittel, Schmiermittel, Viskosifizierungsmittel, Gleitfähigkeitsmittel, Opazifizierungsmittel, Salz und Mischungen davon umfasst.

5. Wässrige Körperpflegezusammensetzung nach Anspruch 1, wobei das Polygalactomannan ausgewählt ist aus der Gruppe bestehend aus Bockshornkleegummi, Guarkernmehl, Tarakernmehl, Johannisbrotkernmehl und Kassiagummi.

6. Wässrige Körperpflegezusammensetzung nach Anspruch 5, wobei das Polygalactomannan Guar umfasst.

7. Wässrige Körperpflegezusammensetzung nach Anspruch 1, wobei Z⁻ ein einfaches Anion, ein Oxoanion oder ein Anion aus einer organischen Säure umfasst.

8. Wässrige Körperpflegezusammensetzung nach Anspruch 1, wobei die Zusammensetzung ferner ein kationisches Polymer umfasst.

9. Wässrige Körperpflegezusammensetzung nach Anspruch 8, wobei das kationische Polymer ausgewählt ist aus der Gruppe bestehend aus kationischer Hydroxyethylcellulose, hydrophob modifizierter kationischer Hydroxyethylcellulose, kationischen Stärken, kationischen Polygalactomannanen, kationischen hydroxyalkylierten Polygalactomannanen und kationischem Diallyldimethylammoniumchlorid.

10. Wässrige Körperpflegezusammensetzung nach Anspruch 9, wobei das kationische Polymer kationische Polygalactomannane umfasst.

11. Wässrige Körperpflegezusammensetzung nach Anspruch 10, wobei das kationische Polygalactomannan kationisches Guar umfasst.

12. Wässrige Körperpflegezusammensetzung nach Anspruch 1, wobei n eine ganze Zahl zwischen 1 und 5 ist.

## Revendications

1. Composition aqueuse de soin personnel, comprenant un conditionneur et un polymère fonctionnalisé par un groupement aminé, où le groupement aminé est pendant et le polymère fonctionnalisé par le groupement aminé répond à la structure suivante : 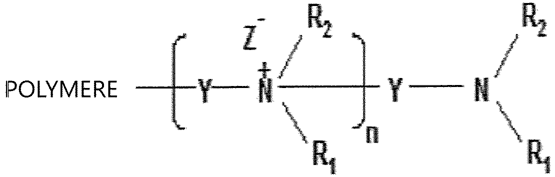 où le polymère comprend un polygalactomannane ayant une pluralité de groupements hydroxyle, où au moins l'un des groupements hydroxyle réagit pour former une liaison oxygène avec Y du groupement aminé, et où le polygalactomannane possède des groupements silanol (-Si-OH) ou silanolate (-Si-O- Na⁺) ; où
Y est un motif alkylène bivalent ou alkylène bivalent substitué ;
R₁ et R₂ sont identiques ou différents et sont hydrogène, un groupement C₁-C₂₀-alkyle, C₆-C₂₀-aryle ou C₇-C₂₀-alkyl (aryl) ;
n est un nombre entier compris entre 1 et 10 ;
Z⁻ est un contre-anion ; et
où le conditionneur comprend une silicone.

2. Composition aqueuse de soin personnel selon la revendication 1, dans laquelle la silicone est choisie dans le groupe constitué par les polyorganosiloxanes, les polyorganosiloxane-polyéther-copolyols, les amodiméthicones, et les matériaux à base de polydiméthylsiloxane cationique.

3. Composition aqueuse de soin personnel selon la revendication 2, dans laquelle la silicone contient un groupement fonctionnel choisi dans le groupe constitué par amino, ammonium, hydroxyle, époxy et polyalkylène glycols.

4. Composition aqueuse de soin personnel selon la revendication 1, où la composition aqueuse de soin personnel comprend en outre au moins un ingrédient supplémentaire choisi dans le groupe constitué par un agent tensioactif, un modificateur de rhéologie, un agent moussant, un agent émulsifiant, un colorant, un parfum, un conservateur, un agent antimicrobien, un agent de blanchiment, un agent lubrifiant, un agent de viscosité, un agent glissant, un opacifiant, un sel, et des mélanges de ceux-ci.

5. Composition aqueuse de soin personnel selon la revendication 1, dans laquelle le polygalactomannane est choisi dans le groupe constitué par la gomme de fenugrec, la gomme guar, la gomme de tara, la gomme de caroube et la gomme de casse.

6. Composition aqueuse de soin personnel selon la revendication 5, dans laquelle le polygalactomannane comprend de la gomme guar.

7. Composition aqueuse de soin personnel selon la revendication 1, dans laquelle Z⁻ comprend un anion simple, un oxo-anion, ou un anion issu d'un acide organique.

8. Composition aqueuse de soin personnel selon la revendication 1, où la composition comprend en outre un polymère cationique.

9. Composition aqueuse de soin personnel selon la revendication 8, dans laquelle le polymère cationique est choisi dans le groupe constitué par l'hydroxyéthylcellulose cationique, l'hydroxyéthylcellulose cationique modifiée de manière hydrophobe, les amidons cationiques, les polygalactomannanes cationiques, les polygalactomannanes hydroxyalkylés cationiques, et le chlorure de diallyldiméthylammonium cationique.

10. Composition aqueuse de soin personnel selon la revendication 9, dans laquelle le polymère cationique comprend des polygalactomannanes cationiques.

11. Composition aqueuse de soin personnel selon la revendication 10, dans laquelle le polygalactomannane cationique comprend de la gomme guar cationique.

12. Composition aqueuse de soin personnel selon la revendication 1, dans laquelle n est un nombre entier compris entre 1 et 5.
